Europäisches Patentamt

European Patent Office

Office européen des brevets

(19)

(11) Publication number: **0 350 887**
**A2**

(12) **EUROPEAN PATENT APPLICATION**

(21) Application number: **89112725.0**

(22) Date of filing: **12.07.89**

(51) Int. Cl.4: **C12N 5/06 , C12N 5/08**

(30) Priority: **12.07.88 JP 174352/88**

(43) Date of publication of application:
**17.01.90 Bulletin 90/03**

(84) Designated Contracting States:
**BE CH DE FR GB IT LI NL SE**

(71) Applicant: **BIOSCIENCE LABORATORY INCORPORATION**
**5-34-5, Shironishi-cho**
**Yamagata-shi Yamagata 990(JP)**

(72) Inventor: **Suzuki, Takahiko**
**2-6-36, Honmachi**
**Sagae-shi Yamagata 991(JP)**
Inventor: **Hoshi, Hiroyoshi**
**5-10-5-C102, Shironishi-cho**
**Yamagata-shi Yamagata 990(JP)**

(74) Representative: **Kolb, Helga, Dr. Dipl.-Chem. et al**
**Hoffmann, Eitle & Partner Patentanwälte**
**Arabellastrasse 4**
**D-8000 München 81(DE)**

(54) Serum-free medium and process for cultivating mammalian cells.

(57) A serum-free medium comprising MCDB107 medium and at least one compound selected from insulin and transferrin, and the medium may contain dexamethasone and/or norepinephrine.
The medium is useful for cultivation of mammalian cells.

EP 0 350 887 A2

## Serum-Free Medium and Process for Cultivating Mammalian Cells

Field of the Invention

The present Invention relates to a serum-free medium and a process for cultivating mammalian cells. More particularly, this Invention relates to a serum-free medium consisting of known ingredients appropriate for cultivation of mammalian cells, especially for cultivation of cardiac muscle cells, and to a process for cultivation of mammalian cells therein.

Prior Arts

Cultivated cardiac muscle cells have recently been used as a study model in many aspects such as growth, differentiation, morphology, physiology, biochemistry and pharmacology of cardiac muscle cells. For cultivating such cardiac muscle cells, ordinary there is used a medium prepared by adding about 10 to 20% of serum to a nutrient medium. However, contents of serum fluctuate widely between lots and also serum contains many unknown components and impurities. These components make an influence over the proliferation of cells to impede analytical study of cardiac muscle cells. For cultivating various mammalian cells, recently development of a serum-free medium consisting of growth factors, hormones, binding proteins, cell adhesion factors, nutrients and other known ingredients has been initiated in place of a serum-containing medium. If the cultivation of cardiac muscle cells can be performed in such a medium consisting of known materials, a great use for elucidation of the mechanism on the action of hormones or drugs over cardiac muscle cells would be attained, and finally an excellent model system for elucidating the pathogenesis of cardiac diseases such as myocardial infarction, stenocardia, myocardiopathy, or myocarditis would be provided. From this point of view, the cultivation of cardiac muscle cells in a serum-free medium has been tried, for example, as shown in the following references.

(1) Mohamed, S.N.W., et al., In Vitro, vol. 19, p. 471 - 478, 1983

This reference reports that pulsating cardiac muscle cells of newborn rat were cultivated in a medium as defined below in a fibronectin treated culture flask over a period of not less than 90 days.
Nutrient medium: [F12: Dullbecco's-ME(1:1)]
Supplemented with hormones, growth factors, etc.:
Insulin (5 $\mu$g/ml), transferrin (5 $\mu$g/ml), selenium (5 ng/ml), fetuin (0.025%), bovine serum albumin (1%), hydrocortisone (5 $\mu$g/ml), $T_4$ (1.0 $\mu$g/ml), EGF (10 ng/ml)

(2) Icekson, G. K. et al., Exp. Cell Res., Vol. 155, p. 113-120, 1984

This reference reports that cardiac muscle cells of newborn rats were cultivated in a medium as defined below in a collagen-treated culture flask over a period of three weeks.
Nutrient medium: [F12: Dullbecco's-ME(1:1)]
supplemented with hormones, growth factors, etc.:
Insulin (5 $\mu$g/ml), hydrocortisone (0.1 $\mu$M), transferrin (25 $\mu$g/ml), fetuin (1 mg/ml)

(3) Nag, A.C. et al., In Vitro Cell & Dev. Biol., Vol., 21, p. 553-562, 1985

This reference reports that cardiac muscle cells of chicken embryo were cultivated in a medium as defined below.
Nutrient medium: F12
Supplemented with hormones, growth factors, etc.:
Fetuin (0.025%,) ascorbic acid (0.02 mg/ml), bovine serum albumin (1%), ECGS (50 $\mu$g/ml), EGF (10 $\mu$g/ml), hydrocortisone (5 $\mu$g/ml), $T_4$ (10 nM) and other ingredients (insulin, transferrin, selenium)
However, the media as described in aforesaid references contain a considerable amount of bovine serum albumin or fetuin ($\alpha$-globulin obtained from bovine serum of embryo or newborn cow) in spite of calling serum-free medium, and are far from the conception of serum-free medium consisting of known materials. Concerning the addition of bovine serum albumin and fetuin, it is considered that bovine serum albumin takes mainly a role as a lipid binding protein to provide cells with necessary lipids, and that, as a partial effect of fetuin, fibronectin contained in this fraction increases to adhere cardiac muscle cells to the culture flask. In this way, such a medium heretofore called a serum-free medium contains certain serum ingredient, and in particular, a medium containing 1% of bovine serum albumin which is equivalent to 20% of serum concentration can not be said to be a serum-free medium at all.

In heart tissues, especially ventricle, there coexist several cells such as vascular endothelial cells, vascular smooth muscle cells or fibroblasts other than cardiac muscle cells. Since serum has a mitogenic effect of these cells, the cultivation in a serum-containing medium already known would stimulate the proliferation of fibroblasts, vascular endothelial cells and vascular smooth muscle cells together with the cultivation of cardiac muscle cells. Therefore, a serum-free medium is essential for selective cultivation of cardiac muscle cells.

Further, the present inventors have found that addition of serum to a medium increases to express the differentiation including rhythmic contraction and that addition of a high concentration of serum exceeding 5% inhibits the DNA synthesis in cardiac muscle cells. Thus, when the DNA synthesis and protein synthesis were examined in a medium containing several concentrations of serum, specially strong inhibition of DNA systhesis was observed at a serum concentration exceeding 5%. At a low serum concentration (2%), cardiac muscle cells were able to survive and culture over a period of long time, and cardiac muscle cells are aggregated by themselves to form myofibrilla, whereby strong rhythmic contraction was observed under an optical microscope. Since some differentiation factor(s) was proved to exist with a substance taken part in DNA synthesis in serum as the result of the above observation, serum containing medium is not appropriate for analytical study of cardiac muscle cells.

## Summary of the Invention

As the result of earnest investigation in view of aforesaid technical aspect, the present inventors have found that mammalian cells, especially cardiac muscle cells can support the long-term survival in a serum-free medium prepared by adding specific known materials to a nutrient medium of MCDB107.

Accordingly, one object of the present invention is to provide a serum-free medium comprising MCDB107 medium and at least one compound selected from insulin and transferrin.

Another object of the present invention is to provide a serum-free medium comprising the above serum-free medium containing at least one compound selected from dexamethasone and norepinephrine.

A further object of this invention is to provide a process for cultivating mammalian cells in the serum-free media mentioned above.

Other objects and advantages of the present invention will be elucidated from the following description.

## Brief Description of the Drawing

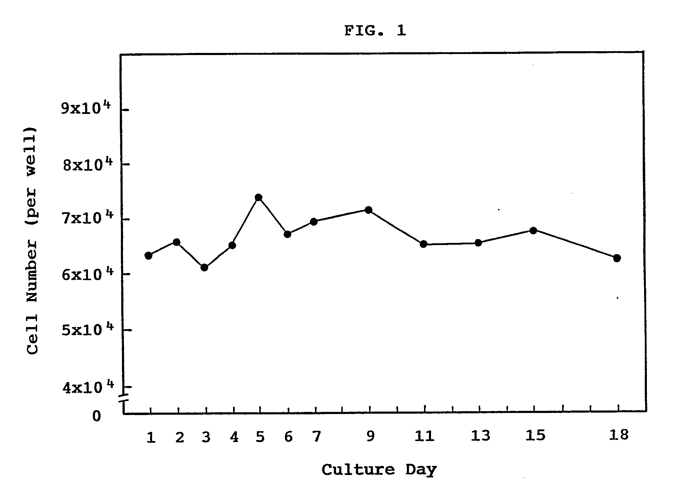

Fig. 1 shows the relation between the culture days and number of cells in the cultivation of cardiac muscle cells of newborn rats in the serum-free medium in the present invention.

## Detailed Description of the Invention

MCDB107 medium used in the present invention is a known medium which is prepared by adding 10 sorts of trace metal containing selenium to F12 medium heretofore used as a nutrient medium and adding HEPES buffer to give a stable pH in a medium [Ref: Mckeehan, W.L. et al., In Vitro, Vol. 16, p. 475-485 (1980)]. This MCDB107 medium had been developed primarily as a serum-free nutrient medium for clonal growth of human fibroblasts in serum-free nutrient medium, but it was proved thereafter that the medium is appropriate as a serum-free nutrient medium for human vascular endothelial cells and smooth muscle cells. This medium was found to be a superior nutrient medium for mammalian cardiac muscle cells. This MCDB107 is commercially available, for example, from Kyokuto, Pharmaceutical Industries Co., Japan.

Serum-free medium of the present invention comprises said MCDB107 medium and at least one compound selected from insulin and transferrin, in which insulin and transferrin have an action to stimulate DNA synthesis and protein synthesis of cardiac muscle cells. Contents of insulin and transferrin in the

EP 0 350 887 A2

medium are not limited particularly, but appropriate amount of insulin is 0.1 to 50 $\mu$g, preferably 1 to 15 $\mu$g and appropriate amount of transferrin is 0.1 to 50 $\mu$g, preferably 0.3 to 20 $\mu$g, more preferably 1 to 15 $\mu$g per ml of MCDB 107 medium. Such a medium containing both insulin and transferrin is preferred, whereby certain synergistic effect of them can be expected. In particular, the medium containing each 10 $\mu$g of insulin and transferrin per ml of MCDB107 medium is preferably appropriate for cultivating cells over a period of long time.

When the serum-free medium of the present invention is to be used in the test of cardiac muscle cells under rhythmic conditions, certain rhythmic stimulants for cardiac muscle cells such as dexamethasone or norepinephrine may be preferably added. Appropriate amount of these rhythmic contraction stimulants for cardiac muscle cells may be adjusted so that concentration of dexamethasone in medium is $10^{-8}$ to $10^{-5}$ M, preferably about $10^{-6}$ M, and concentration of norepinephrine is $10^{-7}$ to $10^{-4}$ M, preferably about $10^{-5}$ M. A medium containing one or both of dexamethasone and norepinephrine in the presence of insulin and transferrin is preferred, showing equal or superior effect to that of serum-containing medium.

Serum-free medium of the present invention can be prepared by adding a prescribed amount of the above-mentioned ingredients to MCDB107 medium, if necessary, followed by sterilization. Addition and sterilization can be performed appropriately in a conventional manner without special limitation. Further other materials in known ingredients may be appropriately added.

Serum-free medium of the present invention can be used similarly to known media. Since it contains no serum ingredient, however, culture surface previously treated with cell adhesion factors such as fibronectin or collagen may be preferably used for stimulating adhesion of cells to culture surface.

Serum-free medium of the present invention is an optimum medium for cultivating cardiac muscle cells of mammals, but it can be used for cultivating other cells of mammals.

Cultivation of mammalian cells in the serum-free medium of the present invention can be performed in a conventional manner such as monolayer culture, stirring culture, tank culture, or hollow fiber culture.

As described in detail above, the serum-free medium of the present invention comprising MCDB107 medium being excellent as a nutrient medium for cultivating cardiac muscle cells and insulin and/or transferrin having an action to stimulate DNA synthesis and protein synthesis can cultivate stably cardiac muscle cells over a period of long time without serum ingredients. Since dexamethasone and norepinephrine have an action to increase rhythmic contraction of cardiac muscle cells, the cell culture system containing them will be an excellent model for investigating the mechanism of rhythmic contraction of cardiac muscle cells.

Since our serum-free, chemically defined medium only contains known ingredients, there is provided with an advantage to easily determine the discovery of a factor stimulating in DNA synthesis or cell proliferation and a differentiation factor inducing rhythmic contraction.

Presently preferred and practical embodiments of the present invention are illustrating shown in the following Reference Examples, Experimental Examples and Examples.

However, these examples are not to be construed to limit the scope of the invention.

## Example

## Reference Example 1

### Isolation of cardiac muscle cells of newborn rat

Cardiac muscle cells of newborn rat used in the following Reference Example and Experimental Examples were isolated in the method below.

Five to 10 newborn Wistar rats of 1 to 5 days old were killed by decapitation. Only ventricular tissues were isolated from the rat's hearts, placed into a petri dish and were minced into fine fragments with scissors. The fine fragments were rinsed two or three times with HEPES buffer to remove the blood cells contaminated from the inside of the ventricle. The tissue fragments were incubated with 0.1% collagenase and 0.05% trypsin at 37°C on a warm bath for 20 to 30 minutes with shaking. After the enzyme treatment, the tissues were gently passed through a No. 100 wire-mesh screen with a 5 ml plastic syringe plunger. Dispersed cell aggregates were rinsed with HEPES buffer to collect the cell suspension in a beaker and centrifuged at 1,200 rpm for 5 minutes. The cell pellet was resuspended in MCDB107 nutrient medium, and the cell suspension was plated in 25 cm$^2$ culture flask. Cardiac muscle cells can be selectively isolated in

4

considering that these cells are less adhesive to the culture surface than vascular endothelial cells and fibroblasts. After cultivation in about 1 hour at 37°C in an atmosphere consisting of 95% air and 5% carbon dioxide in an incubator, the culture flask was taken out and shaken to collect a suspension of cardiac muscle cells. The cell suspension was centrifuged at 1,200 rpm for 5 minutes to give a cell pellet.

Thus collected cardiac muscle cells of newborn rats were used, in any experiments.

Reference Example 2

Effect of fibronectin in the primary culture of cardiac muscle cells of newborn rat

Cardiac muscle cells of newborn rat were suspended in a suitable medium and the number of cells was counted by a hemocytometer. The cardiac muscle cells ($1 \times 10^5$ cells) were seeded into 24-well culture plate pretreated with various concentrations of fibronectin. After the incubation of the cells in MCDB107 nutrient medium for 24 hours, cell spreading ratio was assayed. The cell spreading ratio was determined by photographing each random area of the culture wells with an inverted microscope (x 100 magnification). Approximately 500 cardiac muscle cells were counted. As an index of spreading, round-shaped cells were non-spreading cells, and star- or spindle-shaped cells accurately adhead to the culture surface were spreading cells.

In this experiment, the culture surface pretreated with fibronectin was prepared by adding 1 ml of a solution of fibronectin in various concentrations to 24-well culture plate 1 hour prior to the cell culture and incubating at room temperature for 1 hour.

As the result, fibronectin increased cell spreading of the cardiac muscle cells of newborn rat depending upon its concentration, and very few cell spreading was observed in the absence of fibronectin. Maximum cell spreading activity was observed at about 10 μg/ml of fibronectin, whereby the spreading ratio was about 50%.

Experimental Example 1

Effect of insulin and transferrin on DNA synthesis and protein synthesis in cardiac muscle cells of newborn rat

Cardiac muscle cells of newborn rat were cultivated in MCDB107 nutrient medium for 24 hours. The medium was removed and fresh MCDB107 medium with $^3$H-thymidine (0.5 μCi/ml) and $^{14}$C-leucine (0.05 μCi/ml) and a given amount of insulin (Ins) or transferrin (Tf) was added. Further cultivation was performed for 48 hours. According to a conventional manner, acid-insoluble materials of the cells were prepared and radioactivity of the acid-insoluble materials of the cells was assayed by liquid scintillation counter. Ins- and Tf-free medium (i.e. MCDB107 medium alone) was used as control. The results are shown below in Tables 1 and 2.

As shown in Tables 1 and 2, the addition of insulin and transferrin to the medium increased the incorporation of $^3$H-thymidine and $^{14}$C-leucine into DNA and protein molecules, respectively.

Table 1

| | Concentration (μg/ml) | Incorporation of ³H-thymidine (dpm/10⁵ cells) |
|---|---|---|
| Ins | 0.1 | $12.6 \times 10^3$ |
| | 1 | $22.4 \times 10^3$ |
| | 10 | $23.5 \times 10^3$ |
| Tf | 1 | $15.9 \times 10^3$ |
| Control | | $12.2 \times 10^3$ |

Table 2

| | Concentration (μg/ml) | Incorporation of ³H-thymidine (dpm/10⁵ cells) |
|---|---|---|
| Ins | 0.1 | $8.8 \times 10^2$ |
| | 1 | $10.9 \times 10^2$ |
| | 10 | $12.8 \times 10^2$ |
| Tf | 1 | $8.6 \times 10^2$ |
| Control | | $6.5 \times 10^2$ |

Experimental Example 2

Cultivation of cardiac muscle cells of newborn rat in the serum-tree medium

Cardiac muscle cells of newborn rat ($8 \times 10^4$ cells) were seeded in 24-well culture plate pretreated with fibronectin. MCDB107 containing insulin (10 μg/ml) and transferrin (10 μg/ml) was used as a medium. After the cultivation for a given period of days, the cells were dispersed with trypsin solution (250 μg/ml trypsin solution containing 200 μg/ml EDTA), and cell number was counted by a microcell counter (Toa Iyo Denshi Co., Japan). The result is shown in Fig. 1.

As shown in Fig. 1, cell adhesion rate in 24 hours was 78%. About 24% increase of cell number was observed in 4 to 5th days. Long period cultivation was performed in this medium without reducing cell number for at most 18 days. Cell viability showed always a high value of not less than 90% throughout 18 days.

Experimental Example 3

Effect of various factors on beating cell ratio and beating frequency per minute of cardial muscle cells of newborn rat

Cardiac muscle cells of newborn rat ($10^5$ cells) were cultivated in MCDB107 medium in culture plate for 24 hours. Medium was removed, and fresh MCDB107 medium with insulin (Ins, 10 μg/ml), transferrin (Tf, 1

μg/ml), dexamethasone (Dex, 1 μM) and norepinephrine (NE, 10 μM) singly or in appropriate combination was added. After cultivation for 48 hours, the culture plate was placed on an inverted microscope set to maintain 37°C with a hot plate, and beating cell ratio and beating frequency per minute were measured. For control, the same experiment was carried out in MCDB107 medium containing 2% of fetal calf serum (FCS). The result is shown in Table 3.

As shown in Table 3, addition of dexamethasone alone increased the ratio of beating cell and the beating frequency. This effect was enforced by adding insulin and transferrin. Both ratio of beating cell and beating frequency were increased in the presence of insulin, transferrin and dexamethasone, and beating frequency was markedly increased by adding norepinephrine further. Thus it was shown that the medium containing these materials has the same or more effect as the serum containing medium.

Table 3

|  | Beating cell ratio (%) | Beating frequency (min$^{-1}$) |
|---|---|---|
| MCDB107 alone | 3.9 | 12.0 |
| + Dex | 32.5 | 37.3 |
| + Ins + Dex | 41.9 | 40.5 |
| + Tf + Dex | 35.0 | 51.7 |
| + Ins + Tf + Dex | 80.0 | 104.0 |
| + Ins + Tf + Dex + NE | 95.8 | 254.0 |
| + 2% FCS | 86.6 | 113.0 |

Example 1

In one liter of MCDB107 medium were dissolved each 10 μg/ml of insulin and transferrin. The resulting solution was sterilized by filtration using a membrane filter (pore size: 0.2 to 0.22 μ) to give a serum-free medium.

Example 2

In one liter of MCDB107 medium were dissolved each 10 μg/ml of insulin and transferrin. Further 1 μM of dexamethasone was dissolved therein. The resulting solution was sterilized by filtration using a membrane filter (pore size: 0.2 to 0.22 μ) to give a serum-free medium.

**Claims**

1. A serum-free medium which comprises MCDB107 medium and at least one compound selected from insulin and transferrin.

2. A serum-free medium according to claim 1, wherein said medium contains insulin and transferrin.

3. A serum-free medium according to claim 2, wherein contents of insulin and transferrin in said medium are 10 μg/ml, respectively.

4. A serum-free medium according to claim 1, wherein said medium contains dexamethasone and/or norepinephrine.

5. A process for cultivating mammalian cells which comprises cultivating mammalian cells in a serum-free medium comprising MCDB107 medium and at least one compound selected from insulin and transferrin.

6. A process for cultivating mammalian cells according to claim 5, wherein said serum-free medium contains insulin and transferrin.

7. A process for cultivating mammalian cells according to claim 6, wherein said mammalian cells are cardiac muscle cells.

8. A process for cultivating mammalian cells according to claim 7, wherein said serum-free medium contains dexamethasone and/or norepinephrine.

8

FIG. 1